(19) Europäisches Patentamt
European Patent Office
Office européen des brevets

(11) **EP 2 288 363 B1**

(12) **EUROPEAN PATENT SPECIFICATION**

(45) Date of publication and mention of the grant of the patent:
**18.03.2015 Bulletin 2015/12**

(51) Int Cl.:
**A61K 36/28** (2006.01)       **A61P 31/12** (2006.01)
**A61P 35/00** (2006.01)

(21) Application number: **09746155.2**

(22) Date of filing: **12.05.2009**

(86) International application number:
**PCT/IB2009/005584**

(87) International publication number:
**WO 2009/138860 (19.11.2009 Gazette 2009/47)**

(54) **PLANT EXTRACT AND ITS THERAPEUTIC USE**

PFLANZENEXTRAKT UND DESSEN THERAPEUTISCHE VERWENDUNG

EXTRAIT DE PLANTE ET SON UTILISATION THÉRAPEUTIQUE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **16.05.2008 GB 0808974**
**19.12.2008 CH 19982008**

(43) Date of publication of application:
**02.03.2011 Bulletin 2011/09**

(60) Divisional application:
**14176106.4 / 2 805 725**

(73) Proprietor: **Viridis Pharmaceutical Limited Road Town Tortola (VG)**

(72) Inventor: **KREUTER, Matthias-Heinrich CH-8880 Walenstadt (CH)**

(74) Representative: **Hasler, Erich c/o Riederer Hasler & Partner Patentanwälte AG Kappelestrasse 15 9492 Eschen (LI)**

(56) References cited:
**WO-A-2004/093518       WO-A-2007/057651**
**WO-A-2008/146009       US-B1- 6 300 370**
**US-B1- 6 361 806**

- **DATABASE WPI Week 199637 Thomson Scientific, London, GB; AN 1996-368115 XP002541738 & JP 08 176002 A (KAO CORP) 9 July 1996 (1996-07-09)**

- **DATABASE WPI Week 200528 Thomson Scientific, London, GB; AN 2005-263353 XP002541765 & CN 1 559 482 A (SILAPU A) 5 January 2005 (2005-01-05)**
- **DATABASE WPI Week 200328 Thomson Scientific, London, GB; AN 2003-279512 XP002541768 & CN 1 380 094 A (SLAFU A) 20 November 2002 (2002-11-20)**
- **DATABASE WPI Week 200418 Thomson Scientific, London, GB; AN 2004-186807 XP002541770 & JP 2004 059463 A (LOTTE CO LTD) 26 February 2004 (2004-02-26)**
- **DATABASE WPI Week 200317 Thomson Scientific, London, GB; AN 2003-168423 XP002541771 & CN 1 375 305 A (WANG W) 23 October 2002 (2002-10-23)**
- **ABU-DAHAB R ET AL: "Antiproliferative activity of selected medicinal plants of Jordan against a breast adenocarcinoma cell line (MCF7)" SCIENTIA PHARMACEUTICA 20070930 AT, vol. 75, no. 3, 30 September 2007 (2007-09-30), pages 121-136, XP009121580 ISSN: 0036-8709**
- **RETHY BORBALA ET AL: "Antiproliferative activity of Hungarian Asteraceae species against human cancer cell lines. Part I" PHYTOTHERAPY RESEARCH, vol. 21, no. 12, December 2007 (2007-12), pages 1200-1208, XP009121579 ISSN: 0951-418X**
- **DATABASE WPI Week 200444 Thomson Scientific, London, GB; AN 2004-464566 XP002541742 & JP 2004 175767 A (KOSE KK) 24 June 2004 (2004-06-24)**

**(Cont. next page)**

Remarks:
The file contains technical information submitted after the application was filed and not included in this specification

Remarks:
The file contains technical information submitted after the application was filed and not included in this specification

**Description**

Field of the Invention

This invention relates to a plant extract and to its therapeutic use.

Background of the invention

WO 03/101479 A1 describes the valuable therapeutic properties of a composition comprising various components, typically administered together by intramuscular injection. The composition that was used in the examples comprises a camomile extract, although no direct therapeutic activity is ascribed to it; rather, it is described as an anti-irritant whose presence may alleviate the unpleasant effects of the injection *per se.*

WO 2007/057651 A1 discloses a process for the removal of water-soluble contaminants having lipid groups, especially endotoxins from aqueous compositions of camomile.

In WO 01/13929 A1 is described a substantially pure biologically active extract isolated from *Achillea millefolium*. This extract, preferably a methanolic extract, has antineoplastic activity, and is used as anti-cancer agent.

In WO 2008/146009 A1 are described the effects of camomile essential oil or the seed oil of black cumin or a mixture of both oils on 5-lipoxygenase activity in the human granulocyte cell line HL 60 and on the DNA-synthesis in the human glioblastoma cell line U87MG. In this reference are also described the effects of said oils on the IL-6 release in the human macrophage cell line THP1 and on the DNA-synthesis in the human prostate cancer cell DU 145.

In US 6 300 370 B1 is described a process for manufacturing essential camomile oil. In this process a camomile extraction residue is subjected to a steam distillation or to an aqueous distillation. Said camomile extraction residue is obtained by the treatment of camomile flowers and stalks with an aqueous or organic solvent or a mixture thereof, with or without a preceding steam distillation of the starting material.

P.Vilaginès, P. Delaveau and R. Vilaginès "Inhibition de la replication du poliovirus par un extrait de Matricaria chamomilla (L.)", Comptes Rendus de l'Academie des Sciences, Série III, Tome 301, No. 6, 1985, pages 289 to 294 describe the effect of a hydroalcoholic extract of *Matricaria chamomilla L.* on the growth of poliovirus type 1. When this camomile extract is added during the early stage of poliovirus development, then said extract inhibits cellular and viral RNA synthesis. This inhibition is partially reversible.

Objects of the Invention

It is an object of the present invention to provide a composition for the treatment of an abnormal proliferative and/or viral condition.

It is a further object of the present invention to provide a composition for the synchronization and the S-phase arrest of abnormal proliferative mammalian cells, especially of cancer cells, in the human or animal body.

This synchronization shall comprise the induction of ornithine decarboxylase and/or the inhibition of topoisomerase II.

It is also an object of the present invention to provide a composition for the treatment of an abnormal proliferative condition wherein the treatment comprises the simultaneous or sequential administration of this composition and of at least one anti-tumor agent.

These objects are attained with the present invention.

Summary of the invention

The invention is characterized by the characteristics as defined in the independent claims.

Preferred embodiments are defined in the dependent claims.

This composition may also consist of an organic extract of at least one *Chamomilla* plant and of at least one anti-tumor agent, and occasionally of at least one pharmaceutical and/or dermatological acceptable auxiliary agent.

Surprisingly, it has now been found that a camomile extract, obtained from the flower heads, such as that described in WO 2007/057651 A1, has valuable therapeutic properties. In particular, it has been found that it can reduce the DNA-and RNA-synthesis without substantial effect on protein synthesis, from which utility in the treatment of cancer may be deduced.

Even more surprisingly it has been found that organic extracts of the tubular flowers of *Matricaria recutita L.* (Flores tubiformis) are suitable for the synchronization and the S-phase arrest of abnormal proliferative mammalian cells, especially of cancer cells.

This synchronization takes place due to the induction of ornithine decarboxylase (transfer from $G_0$-phase into $G_1$-phase) and the inhibition of topoisomerase II (accumulation and arrest in the early S-phase).

It was also found that the inhibition of topoisomerase II was more than 100-fold stronger with an organic extract than with an aqueous extract (with respect to the concentration for complete inhibition of the enzyme).

Due to the fact that the inhibition of the topoisomerase II is crucial for the effectiveness of cell synchronization the organic extracts of the tubular flowers of *Matricaria recutita L.,* (Flores tubiformis) of the present invention are much more potent than the corresponding aqueous extracts.

Description of the Invention

The invention is based on data obtained using an aqueous extract of camomile and on data obtained using organic extracts, especially alcoholic extracts, for example ethanolic extracts, of the tubular flowers of *Matricaria recutita L.,* (Flores tubiformis).

A composition comprising an organic extract is claimed. However, for purposes of comparison parts of the original disclosure relating to aqueous extracts are retained.

The available evidences show that the aqueous camomile extract obtained as described below maintains protein biosynthesis, whereas DNA- and RNA-biosynthesis is reduced. This is a good measure of the desirable properties of this extract.

The aqueous and/or organic extracts may be obtained by any suitable procedure known to those of ordinary skill in the art. The extracts may be obtained by using an aqueous and/or organic medium, especially an alcoholic medium, such as an ethanolic medium, and separated from other components.

A preferred procedure for the preparation of an aqueous extract is described in WO 2007/057651A1.

Preferably, the composition according to the present invention additionally comprises at least one component selected from the group consisting of pharmaceutical aids, preferably selected from the group consisting of pharmaceutical agents and pharmaceutical excipients.

The aqueous extract may be subjected to a purification process. Such an extract comprises a multi-component mixture of water-soluble components. It may be obtained by adding water to the appropriate plant part, to obtain a suspension that is then usually heated to a temperature below the boiling point of water, e.g. 90-94°C, and then cooled to room temperature.

The aqueous extract is then subjected to two filtration steps. For the purposes of illustration only, these will be described below as microfiltration and ultrafiltration, respectively. Other techniques, such as use of a lipophilic barrier, may be suitable. Each filtration step may be conducted in one, two or more than two stages, if desired.

Microfiltration is applied in order to remove material that would otherwise compromise the effectiveness of the ultrafiltration step.

In the following part are described possible embodiments of the present invention as well as for purposes of comparison parts of the original disclosure relating to aqueous extracts are retained.

The analysis of bacterial endotoxins of the samples obtained in the Examples was performed with the Cambrex PyroGene assay using a dilution factor of 1:10.000.

**Examples 1 and 2**

45 g of yellow tubular camomile flowers (*Chamomilla recutita*) were mixed with 900 g of water (Aqua purificata, Ph. Helv.). This mixture was heated to a temperature between 90°C and 94°C within 20 to 30 minutes. Thereafter the mixture was stored at room temperature (15°C to 25°C) until a temperature between 30°C and 35°C was reached.

The drug residue was removed by deep layer filtration. The obtained crude filtrate was clarified by filtration through a 0.22 $\mu$m membrane.

To the clarified filtrate, 0.3% (Example 1) or 0.1 % (Example 2), with respect to the extract mass, of ricinus oil (Ph.Eur.) was added. The obtained mixture was homogenized for 5 minutes. This so prepared extract was filtered (in tangential flow mode) with retentate recovery via a 0.22 $\mu$m membrane.

The obtained permeate was filtered (in tangential flow mode) with retentate recovery via a 0.1 $\mu$m membrane. Finally, the obtained permeate was filtered (in tangential flow mode) with retentate recovery via a 1000 kDa membrane.

Residue of bacterial endotoxins in each final filtrate: < 100 EU/ml.

**Examples 3 to 5**

Example 1 was repeated, except that, instead of ricinus oil, 0.3% (Example 3), 1.0% (Example 4; VIP-E_Matr'06_1003) and 3.0% (Example 5), with respect to the extract mass, of mygliol (Ph. Eur.) was added to the clarified filtrate.

Residue of bacterial endotoxins in each final filtrate: < 100 EU/ml.

**Example 6**

This Example uses a revised protocol, in which heating and cooling were performed, not in an autoclave but in a 10 L double layer vessel under stirring (max. temperature of heating device 140°C).

Mygliol was added instead of ricinus oil. The mygliol was "Mygliol 812 for parenteral use" from Hanseler. The mixture was stirred at room temperature for 10 minutes, instead of homogenization.

Microfiltrations according to the above described process were all performed with Millipore Pellicon 2 systems. For better practicability and to avoid time-consuming cleaning procedures, the microfiltrations in this Example were performed with the following equipment:

| Filtration | Filter System | Cartouche |
|---|---|---|
| 0.2 $\mu$m filtration | Millipore Pellicon 2 | Durapore 0.2 $\mu$, C-screen |
| 0.1 $\mu$m filtration | One way filter | Millipack 200, 0.1 $\mu$m |
| 1000 kDa filtration | Millipore Pellicon 2 | Biomax 1000 kDa, V-Screen |
| 0.2 $\mu$m filtration | One way filter | Millipack 200, 0.2 $\mu$m |

In addition, phenol was added, for stabilization of the extract. The amount of added phenol was 6.0-8.0 mg/ml. It was added after the 1000 kDa filtration. After the addition, the suspension was stirred for approximately 10 minutes, until all phenol was dissolved.

The endotoxin level was low in each case.

**Example 7** (Preparation of a liquid extract ViP-E_Matr'08_1102) (Inventive Extract)

100 g of tubular flowers from the inflorescence of *Matricaria recutita L.* were extracted under stirring at a temperature between 40°C and 60°C during 2 hours with 500 g 80% (m/m) ethanol, corresponding to a drug to solvent ratio of 1/5. Subsequently the preparation was subjected to a deep layer filtration using a cellulose filter (AF 6 Filtrox®). 385 g of a clear dark brown liquid extract with a solid content of 4.43 % (m/m) were obtained.

**Example 8** (Preparation of a liquid extract ViP-E_Matr'08_1106) Inventive Extract)

100 g of tubular flowers from the inflorescence of *Matricaria recutita L.* were extracted under stirring at a temperature between 40°C and 60°C during 2 hours with 500 g 90% (m/m) ethanol, corresponding to a drug to solvent ratio of 1/5. Subsequently the preparation was subjected to a deep layer filtration using a cellulose filter (AF 6 Filtrox®). 398 g of a clear dark brown liquid extract with a solid content of 1.89 % (m/m) were obtained.

**Example 9** (Preparation of a liquid extract ViP-E_Matr'08_1105) Inventive Extract)

100 g of tubular flowers from the inflorescence of *Matricaria recutita L.* were extracted under stirring at a temperature between 40°C and 60°C during 2 hours with 500 g 99.9% (m/m) ethanol, corresponding to a drug to solvent ratio of 1/5. Subsequently the preparation was subjected to a deep layer filtration using a cellulose filter (AF 6 Filtrox®). 412 g of a clear dark brown liquid extract with a solid content of 1.54 % (m/m) were obtained.

The following examples illustrate the pharmacological activity profile of the extracts of examples 4, 7, 8 and 9.

**Example 10** (Induction of ornithine decarboxylase expression)

With the liquid extract VIP-E_Matr'06_1003 prepared according to example 4 were carried out ornithine decarboxylase expression experiments.

For the measurement of the induction of ornithine decarboxylase expression this extract was added to HepG2 cells in concentrations of 150, 100, or 30 $\mu$g/ml. The so treated cells were cultivated during 6 hours, 24 hours, or 48 hours in 10% FBS culture medium.

Then was determined the change of the amount of the ornithine decarboxylase by means of the Westernblot analysis.

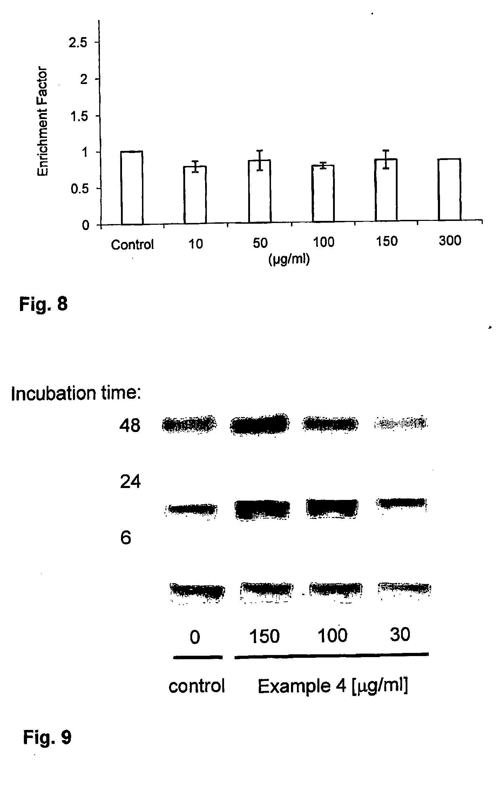

It is obvious from the data shown in Figure 9 that the extract according to the present invention induces the ornithine decarboxylase expression in a concentration-dependent manner.

**Example 11** (comparative example; Inhibition of topoisomerase I activity)

With the liquid extract ViP-E_Matr'08_1102 prepared according to example 7 were carried out topoisomerase I activity experiments with extract concentrations of 0.3, 1, 3, 10, or 30 μg/ml. As positive control camptothecin was carried along.

For the measurement of the inhibition of topoisomerase I activity this extract was added to purified human DNA topoisomerase I and the "Topoisomerase I Drug Screening Kit" from TopoGEN. Thereby it was proceeded according to the manufacturer's protocol. The DNA was separated by electrophoresis on an agarose gel and visualized under UV light after staining with ethidium bromide. With this approach the relaxed DNA (topoisomers) generated by topoisomerase I could be detected and clearly separated from the supercoiled DNA substrate.

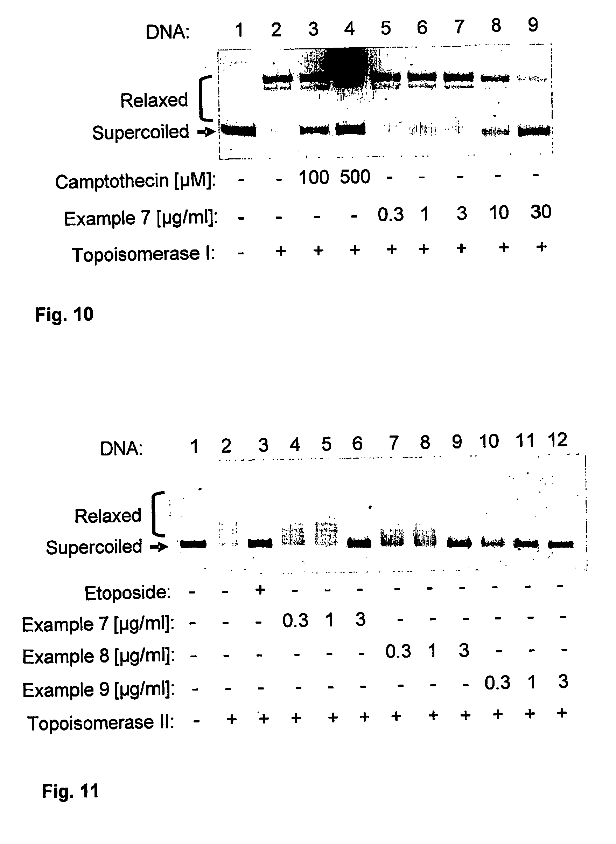

It is obvious from the data shown in Figure 10 that the extract according to the present invention inhibits topoisomerase I activity only very weak but in a concentration-dependent manner.

**Example 12** (Inhibition of topoisomerase II activity)

With the liquid extract ViP-E_Matr'08_1102, prepared according to example 7, the liquid extract ViP-E_Matr'08_1106, prepared according to example 8, or the liquid extract ViP-E_Matr'08_1105, prepared according to example 9, were carried out topoisomerase II activity experiments with extract concentrations of 0.3, 1, or 3 μg/ml. As positive control etoposide was carried along.

The inhibition of topoisomerase II was determined using purified human DNA topoisomerase IIα and the "Topoisomerase II Drug Screening Kit" from TopoGEN. Thereby it was proceeded according to the manufacturer's protocol. The DNA was separated by electrophoresis on an agarose gel and visualized under UV light after staining with ethidium bromide. With this approach the relaxed DNA (topoisomers) generated by topoisomerase II could be detected and clearly separated from the supercoiled DNA substrate.

It is obvious from the data shown in Figure 11 that all of the extracts according to the present invention strongly inhibit the topoisomerase II activity in a concentration-dependent manner. The extent of inhibition clearly increases with increasing concentrations of ethanol % m/m as extraction solvent (99 %>90%>80%). The extract prepared with 99 % m/m ethanol showed a practically complete inhibition of the enzyme activity even at a concentration as low as 300 ng/ml.

This inhibition was more than 100-fold stronger than the inhibition obtained with the aqueous extract of example 4. Nearly 150 μg/ml were necessary for complete inhibition (data not shown).

**Example 13** (Induction of cell cycle arrest in S-phase)

With the liquid extract VIP-E_Matr'06_1003 prepared according to example 4 were carried out cell cycle analysis experiments. As positive control for cell cycle arrest in S-phase camptothecin was carried along.

For the measurement of the induction of cell cycle arrest this extract was added to HepG2 cells in concentrations of 10, 50, 100, or 150 μg/ml. The so treated cells were cultivated during 48 hours in 10% FBS culture medium.

Then was determined the change of the amount of cells in G1-, S- or G2-phase of the cell cycle by means of the cell cytometry analysis.

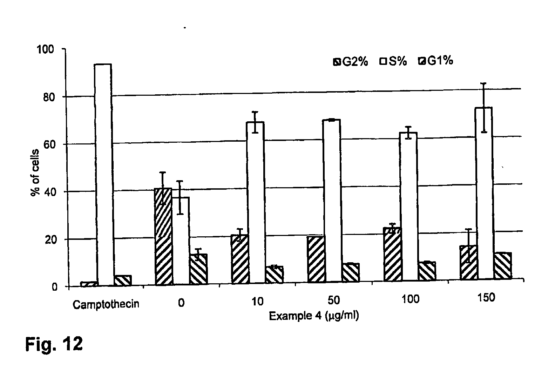

It is obvious from the data shown in Figure 12 that the extract according to the present invention induces a strong cell cycle arrest in S-phase already after 48 hours of incubation at concentrations as low as 10 μg/ml.

**Example 14** (Preparation of a resinous extract)

From a liquid extract obtained in analogy to example 9 the solvent was evaporated under reduced pressure (300 to 10 mbar) and at slightly elevated temperature (35°C to 45°C). 6.4 g of a dark brown resinous extract with a dry material content of >99 % (m/m) were obtained.

The obtained resinous extract was found to be free or nearly free of essential oils (< 0.01% m/m). The content of ethanol was found to be < 0.05 % (m/m), and the content of water was found to be < 0.01% (m/m).

**Example 15** (Preparation of a water-free liquid extract)

To 6.4 g of a liquid extract obtained in analogy to example 9 were added 6.4 g of oleic acid (Ph.Eur.) and 19.2 g of Macrogol 400 (Ph.Eur.).

The ethanol was evaporated under reduced pressure (300 to 10 mbar) and at slightly elevated temperature (35°C to 45°C). 31.8 g of a dark brown free-flowing liquid extract with a content of non-volatile components of >99 % (m/m) were obtained.

The obtained water-free liquid extract was found to be free or nearly free of essential oils (< 0.01% m/m). The content of ethanol was found to be < 0.05 % (m/m), and the content of water was found to be < 0.01% (m/m).

**Example 16** (Preparation of a water-free liquid extract)

To 10 g of the resinous extract obtained in analogy to example 14 were added 10 g of oleic acid (Ph.Eur.) and 20 g of Macrogol 400 (Ph.Eur.).
This mixture was homogenized.
The so obtained water-free liquid extract had a content of non-volatile components of >99 % (m/m).

**Example 17**

20 g of yellow tubular camomile flowers (*Anthemis nobilis L.*) were mixed with 380 g of water (Aqua purificata, Ph. Helv.). This mixture was heated to a temperature between 90°C and 94°C within 20 to 30 minutes. Thereafter the mixture was stored at room temperature (22°C) until a temperature between 30°C and 35°C was reached.
The drug residue was removed by deep layer filtration. The obtained crude filtrate was clarified by filtration through a 0.22 $\mu$m membrane.
To the clarified filtrate, 0.3% mygliol (Ph. Eur.), with respect to the extract mass, was added. The obtained mixture was homogenized for 5 minutes. This so prepared extract was filtered (in tangential flow mode) with retentate recovery via a 0.22 $\mu$m membrane.
The obtained permeate was filtered (in tangential flow mode) with retentate recovery via a 0.1 $\mu$m membrane. Finally, the obtained permeate was filtered (in tangential flow mode) with retentate recovery via a 1000 kDa membrane.
There were obtained 309 g of a clear, aqueous extract with a dry material content of 1.1 % m/m.

**Example 18**

Same procedure as in Example 17 but 20 g of the whole flower heads of *Anthemis nobilis L* were used.
There were obtained 295 g of a clear, aqueous extract with a dry material content of 1.6 % m/m.

**Example 19** (Inventive Extract)

20 g of tubular flowers from the inflorescence of *Anthemis nobilis L.* were extracted under stirring at a temperature between 40°C and 60°C during 2 hours with 100 g 99.9% (m/m) ethanol, corresponding to a drug to solvent ratio of 1/5. Subsequently the preparation was subjected to a deep layer filtration using a cellulose filter (AF 6 Filtrox®). 71 g of a clear dark brown liquid extract with a solid content of 1.62 % (m/m) were obtained.

**Example 20** (DNA synthesis (BrdU Incorporation Assay)

The liquid extract according to Example 19 was examined on its ability to inhibit DNA synthesis in human hepatocellular carcinoma cells (HepG2) *in vitro.*
To determine cellular proliferation, freshly synthesized DNA was quantified using the 5-Bromo-2'-deoxy-uridine (BrdU) Labeling and Detection Kit III (Roche Applied Science; Mannheim, Germany). BrdU is a thymidine analog which would be incorporated into new cellular DNA. [See: Gratzner, H. G. (1982) Science 218, 474-475].
In short, human hepatocellular carcinoma cells (HepG2) were harvested by trypsinisation and seeded at 10'000 cells/well in 96 well micro-plates. After 24 hours of pre-incubation at 37°C and 5% $CO_2$, the cells were treated with the liquid extract according to Example 19 at the following concentrations for 30 hours: 0, 1, 3, 10 and 100 $\mu$g/ml.
10 $\mu$l of BrdU (100$\mu$M) were then added to the cells and further incubated for 18 hours. After incubation, the cells were washed 3 times with culture media to remove the excess BrdU before being fixed with ethanol. Prior to incubation with the anti-BrdU antibody, labeled with peroxidase (POD), DNA was partially digested with nucleases to allow the antibody to access the incorporated BrdU. After washing the excess antibody, the POD substrate ABTS was added. POD catalyzes the cleavage of ABTS, producing a coloured reaction product. The absorbance of the reaction product was measured at 405 nm (reference wavelength at 490 nm) with a Satire multifunctional microplate reader (Tecan, Mannedorf, Switzerland). The measured absorbance per well correlates directly to the level of BrdU incorporated into cellular DNA.
Sample points were measured as triplicates; errors were expressed as standard deviation (data no shown).
The data of the samples were expressed as a percentage of the solvent control values, and the $IC_{50}$ values were calculated using GraphPad Software Inc (Prism, version 5, San Diego, CA. USA).

Results

The results showed that the liquid extract according to Example 19 had an $IC_{50}$ value of approximately 7 $\mu$g/ml (6.228 to 7.881 $\mu$g/ml). Thus, said liquid extract inhibited the growth of human hepatocellular carcinoma cells (HepG2) in a dose dependent manner.

Compositions according to the invention can be formulated by methods known to those skilled in the art. Pharmaceutically acceptable components should be used.

Administration is preferably by intravenous or, more preferably, intramuscular injection.

The pharmaceutical composition containing the active ingredient may be also in a form suitable for oral use.

Therapeutic use involves the treatment (and possibly also the prevention) of cancer, for example lung cancer, liver cancer and cancer of the pancreas. Other uses include topical conditions such as psoriasis, scleroderma and pemphigus, infectious bronchitis, cancers including sarcomas (such as Kaposi's sarcoma), leukemia, skin cancer and the carcinomas whose treatment is specifically illustrated below, as well as AIDS. More generally, the composition may be used for therapy of proliferative and viral conditions, especially those associated with DNA- or RNA-viruses. The action on RNA-viruses may be direct, while the action on DNA-viruses and cancer at least may be progressive. The medicament may also be useful in therapy of other genetic disorders such as motor neurone diseases and multiple sclerosis.

The medicament can also be used to treat other viral conditions. For example, the virus may be a coronavirus, as in the case of SARS (severe acute respiratory syndrome). Further, the medicament may have utility in veterinary medicine, e.g. in fowl's diseases such as Newcastle disease and fowl pox.

The following study illustrates further aspects of the invention obtained with the extract according to example 4.

In the following part is given a short description of the Figures:

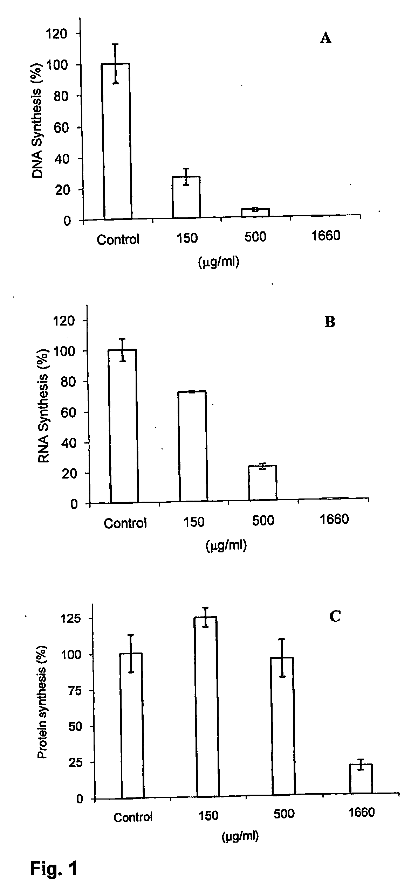

Figure 1 relates to System 4 and shows the effect of the extract according to example 4 on the DNA-synthesis (A), on the RNA-synthesis (B), and on the protein biosynthesis (C) in HepG2 cells. The used extract concentrations are 150, 500, and 1660 $\mu$g/m).

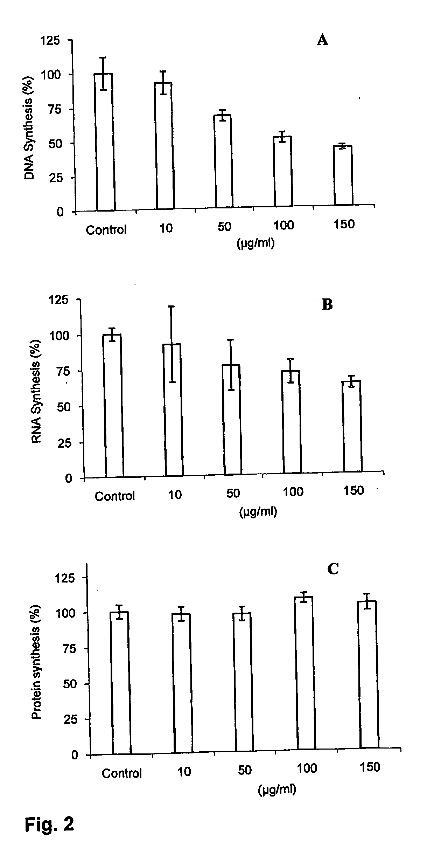

Figure 2 is in analogy to Figure 1, but the used extract concentrations are 10, 50, 100, and 150 $\mu$g/ml.

Figure 3 relates to System 4 and shows the effect of the extract according to example 4 on RNA-synthesis (A) and proteinbiosynthesis (B), calculated in consideration of the results shown in Figure 2(A).

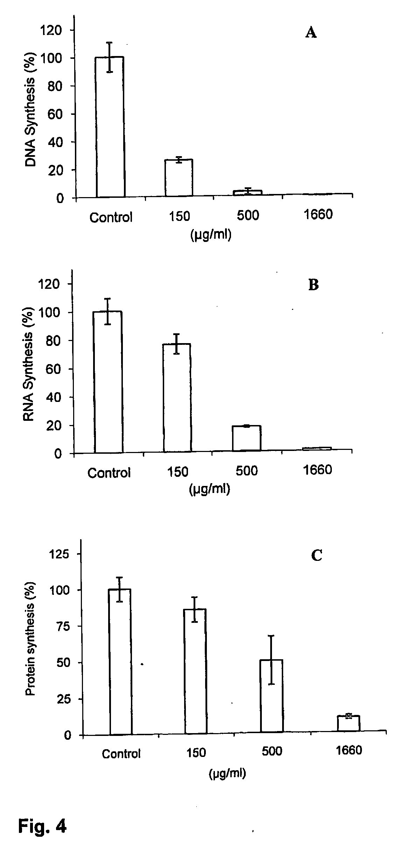

Figure 4 relates to System 4 and shows the effect of the extract according to example 4 on the DNA-synthesis (A), on the RNA-synthesis (B), and on the proteinbiosynthesis (C) in HT1376 cells. The used extract concentrations are 150, 500, and 1660 $\mu$g/ml.

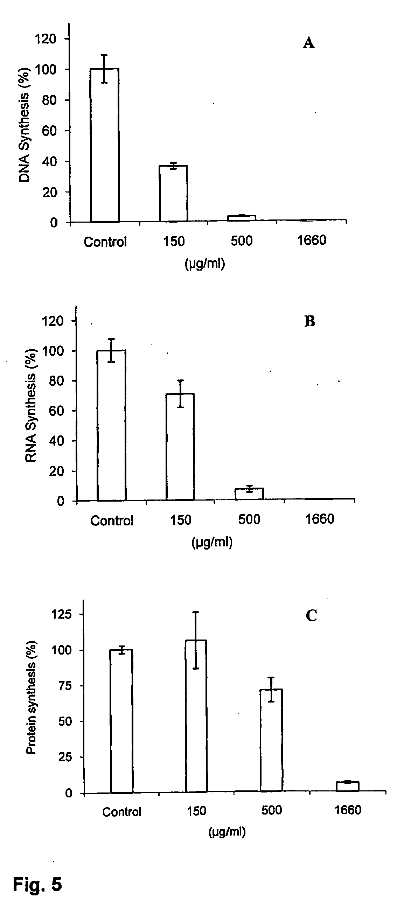

Figure 5 relates to System 4 and shows the effect of the extract according to example 4 on the DNA-synthesis (A), on the RNA-synthesis (B), and on the proteinbiosynthesis (C) in C33-A cells. The used extract concentrations are 150, 500, and 1660 $\mu$g/ml.

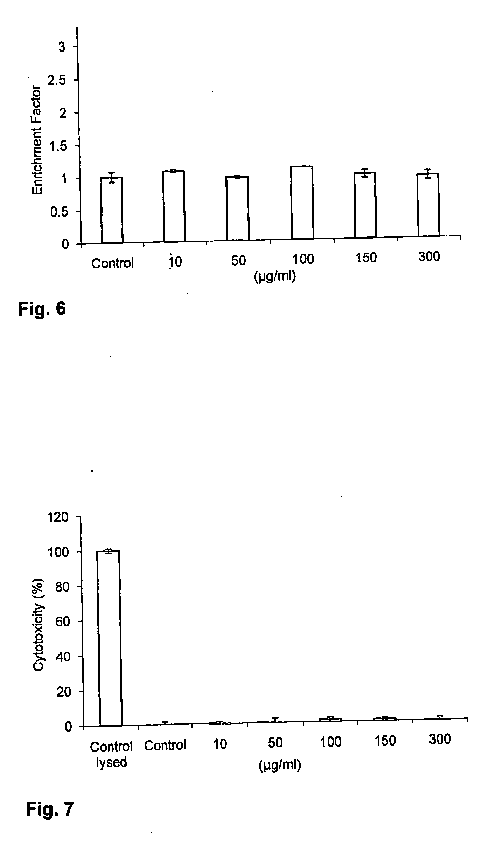

Figure 6 relates to System 5 and shows the effect of the extract according to example 4 on the induction of the apoptosis in HepG2 cells after 24 hours. The used extract concentrations are 10, 50, 100, 150, and 300 $\mu$g/ml.

Figure 7 relates to System 5 and shows the effect of the extract according to example 4 on the cytotoxicity (membrane integrity) in HepG2 cells after 24 hours. The used extract concentrations are 10, 50, 100, 150, and 300 $\mu$g/ml.

Figure 8 relates to System 5 and shows the effect of the extract according to example 4 on the induction of the apoptosis in HepG2 cells after 48 hours. The used extract concentrations are 10, 50, 100,150, and 300 $\mu$g/ml.

Figure 9 relates to example 10 and shows the effect of the extract according to example 4 on the induction of the ornithine decarboxylase expression in HepG2 cells after 6, 24, and 48 hours. The used extract concentrations are 30, 100, and 150 $\mu$g/ml.

Figure 10 relates to example 11 and shows the effect of the extract according to example 7 on the inhibition of topoisomerase I in a cell-free assay. The used extract concentrations are 0.3, 1, 3, 10, and 30 $\mu$g/ml. As positive control camptothecin was used.

Figure 11 relates to example 12 and shows the effect of the extracts according to examples 7, 8, and 9 on the inhibition of topoisomerase II in a cell-free assay. The used extract concentrations are 0.3, 1, and 3 $\mu$g/ml. As positive control etoposide was used.

Figure 12 relates to example 13 and shows the effect of the extract according to example 4 on the induction of cell cycle arrest in S-phase in HepG2 cells. The used extract concentrations are 10, 50, 100, and 150 $\mu$g/ml. As positive control camptothecin was used.

Figure 13 relates to example 12 and shows the effect of the extract according to example 7 on the inhibition of topoisomerase II in a cell-free assay. The used extract concentrations are 0.3, 3, 3, 10, and 30 $\mu$g/ml. As positive control etoposide was used. For comparison reasons 0.3, 3, and 30 $\mu$g/ml of $\alpha$-bisabolol or 0.3, 3, and 30 $\mu$g/ml of the essential oil of *Matricaria recutita L.* was used.

**1) Aim of the study**

System 4: RNA-, DNA- and Proteinbiosynthesis

The influence of the extract according to example 4 on RNA-, DNA- and proteinbiosynthesis using three different cell lines (HepG2: liver carcinoma cells; C33-A: cervix carcinoma cells and HT1376: bladder carcinoma cells) was examined *in vitro.*

System 5: Apoptosis and Membrane Integrity

Additionally the influence of the extract according to example 4 on the induction of apoptosis was tested on HepG2 cells. At the same time the cytotoxicity (membrane integrity) of the extract according to example 4 was examined on HepG2 cells.

**2) Material and Methods**

*2.1) Samples and sample preparation*

| Extract | Description | Origin | Lot | Extract Type |
|---|---|---|---|---|
| VIP-E_Matr'06_1003 | *Matricaria recutita* tubular flowers | Pentapharm | 578-01/End; 7.20.2006 | Aqueous |

VIP-E_Matr'06_1003 was prepared according to example 4.

*2.2) Assay conditions*

| Assay | Cell lines | Incubation time | Sample | Sample concentration |
|---|---|---|---|---|
| RNA-Synthesis | HepG2 C33-A HT1376 | 24h | VIP-E_Matr'06_1003 | 150/500/ 1660$\mu$g/ml 10/50/100/ 150$\mu$g/ml |
| DNA-Synthesis | HepG2 C33-A HT1376 | 24h | VIP-E_Matr'06_1003 | 150/500/ 1660$\mu$g/ml |
| Proteinbiosynthesis | HepG2 C33-A HT1376 | 24h | VIP-E_Matr'06_1003 | 150/500/ 1660$\mu$g/ml |
| Apoptosis | HepG2 | 24h | VIP-E_Matr'06_1003 | 10/50/100/150/ 300$\mu$g/ml |
| Apoptosis | HepG2 | 48h | VIP-E_Matr'06_1003 | 10/50/100/150/ 300$\mu$g/ml |
| Cytotoxicity (membrane integrity) | HepG2 | 24h | VIP-E_Matr'06_1003 | 10, 50, 100, 150, 300$\mu$g/ml |

*2.3) Assays*

2.3.3) RNA-/DNA-Synthesis

For the RNA- and DNA-synthesis, assay cells (HepG2: hepatocellular carcinoma, human; C33-A: cervical carcinoma, human and HT1376: bladder carcinoma, human) were harvested by trypsinisation and seeded at 10'000 cells/well in a 96 well plate. After treatment of the cells with the samples at the required concentrations the plate was incubated for 2 hours at 37°C and 5% $CO_2$. The cells were pulsed with 5-$^3$H-Uridine (1$\mu$Ci/ml) (Perkin Elmer) for the RNA-synthesis and with 6-$^3$H-Thymidine (1$\mu$Ci/ml) (Perkin Elmer) for the DNA-synthesis during a further incubation period of 24 hours. The cells were washed with PBS and fixed twice with methanol for 5 min. The protein was precipitated by 0.3N TCA. After a washing step 150$\mu$l 0,3N NaOH was added for 15min to lyse the cells. Negative controls t(0) were carried out with the samples without cells.

To detect the incorporated 5-$^3$H-Uridine for the RNA-synthesis and the 6-$^3$H-Thymidine for the DNA-synthesis the samples were transferred in scintillation tubes with scintillation cocktail. The quantification was performed in a Tri-Carb 1900 TR liquid scintillation counter (Packard, USA).

The effect of several concentrations of samples was measured by determining amount of radiolabel (dpm) under the assay conditions. Dose related values were expressed as a percentage of the positive control values. Sample points were measured as duplicates, errors are expressed as difference from the mean.

The specific cellular 5-$^3$H-Uridine incorporation ratio (%) was calculated in consideration of the results from the DNA-synthesis assay. The respective synthesis values (%) were divided by the DNA-synthesis coefficient.

2.3.4) Proteinbiosynthesis

For the proteinbiosynthesis assay cells (HepG2: hepatocellular carcinoma, human; C33-A: cervical carcinoma, human and HT1376 : bladder carcinoma, human) were harvested by trypsinisation and seeded at 10'000cells/well in a 96 well plate. After treatment of the cells with the samples at the required concentrations the plate was incubated for 2 hours at 37°C and 5% $CO_2$. The cells were pulsed with L-[3,4,5-$^3$H(N)]-Leucine, (1$\mu$Ci/ml) (Perkin Elmer) during a further incubation period of 24 hours. The cells were washed twice with PBS, lysed with RIPA-buffer and incubated on ice for 15min. The lysate was transferred in a tube. 250$\mu$l ice cold TCA (20%) was added and the samples were incubated on ice for further 15min. After centrifugation for 20min at 10'000xg and 4°C the supernatant was removed and the pellet was resuspended in 250$\mu$l TCA (5%) and centrifuged again. The received lysates of each sample were resuspended in 250$\mu$l NaOH (0,2N) and heated for 2 min at 50°C. Negative controls t(0) were carried out with the samples without cells.

To detect the incorporated L-[3,4,5-3H(N)]-Leucine the samples were transferred in scintillation tubes with scintillation cocktail. The quantification was performed in a Tri-Carb 1900 TR liquid scintillation counter (Packard, USA).

The effect of several concentrations of the sample was measured by determining the amount of radiolabel (dpm) under the assay conditions. Dose related values were expressed as a percentage of the positive control values. Sample points were measured as quadruplicates, errors are expressed as standard deviations.

The specific cellular L-[3,4,5-3H(N)]-Leucine incorporation ratio (%) was calculated in consideration of the results from the DNA-synthesis assay. The respective synthesis values (%) were divided by the proliferation coefficient.

2.3.5) Apoptosis assay

HepG2 cells (hepatocellular carcinoma, human) were harvested by trypsinisation and seeded at 10'000 cells/well in 100$\mu$l in a 96 well plate. After 24h, 20$\mu$l samples and 80$\mu$l of fresh medium (total volume 200$\mu$l) were added. The plate was further incubated for 24 hours and in an additional experiment for 48hours. After the centrifugation of the plate for 10min at 200xg, the supernatant was discarded and the pellet was lysed for 30 min at room temperature in 200 $\mu$l Lysis buffer. The lysate was finally centrifuged for 10min at 200xg. The solvent controls (= negative controls) were performed with solvent instead of sample. Seeding and incubation were performed in an incubator at 37°C and 5% $CO_2$.

The cytoplasmatic histone associated DNA-fragments (mono- and oligonucleosomes) in lysate supernatant were determined *in vitro* with a Cell Death Detection ELISA$^{plus}$ Kit (Roche, Cat-No: 11 774 425 001) according to the supplier recommendation.

The absorbance was measured with a microplate reader device (TECAN Infinite M200) at a wavelength of $\lambda$ = 405nm and a reference wavelength of A = 490nm. The specific enrichment factor of mono- and oligonucleosomes released into the cytoplasm was calculated as following:

$$\text{Enrichment factor} = \text{absorbance sample/absorbance of the corresponding negative control}$$

Sample points were measured as duplicates, errors are expressed as difference from the mean.

2.3.6) LDH-Cytotoxicity assay (Induction of necrosis)

LDH-Cytotoxicity assay (membrane integrity/induction of necrosis): the cytotoxicity was tested on HepG2 cells (hepatocellular carcinoma, human). The cells were seeded in a 96 well plate at 10'000 cells per well over night. 20 $\mu$l

of the respective samples and solvent controls at the indicated concentrations and 80 μl of fresh medium (total volume 200 μl) were added. Seeding and incubation were performed in an incubator at 37°C and 5% $CO_2$. After an incubation period of 24 hours, the membrane integrity was measured with a LDH-cytotoxicity Assay Kit (BioVision, #K311-400, CA USA).

The membrane integrity of the cells was determined by measuring the activity of cytosolic lactate dehydrogenase (LDH) released into the medium under the influence of samples. The activity of the enzyme was measured by determining the formazan produced from the tetrazolium salt INT as substrate. The quantity of formazan was measured directly by determining the optical density (OD) with a plate reader (TECAN Infinite M200) at a wavelength of λ=490 nm.

For each test concentration, the OD values of the background (assay mixture with samples but without cells) was subtracted from OD measurements with cells. $OD_{490}$-values were transformed into percentage values with cytotoxicity readings of 100% corresponding to measurements of the cells lysed (triton X-100) with solvent, and cytotoxicity readings of 0% corresponding to cells incubated with solvent only. The optical measurements were performed as duplicates. Errors were expressed as difference from the mean.

## 3) Summary

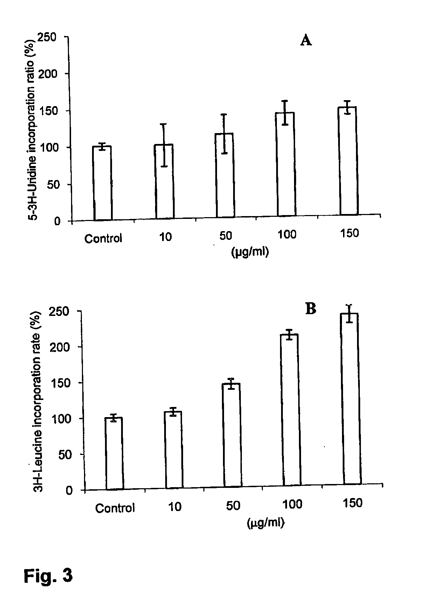

With respect to the DNA-, RNA- and proteinbiosynthesis the extract according to example 4 showed the following effects: the DNA-synthesis is dose dependent suppressed, while up to considerably high doses the RNA-synthesis is still intact and the proteinbiosynthesis is only marginal affected or, if adapted to the amount of proliferating cells, even increased (Figure 3). This effect is evident for all three cell lines in non-cytotoxic dosage ranges and can be interpreted as a sign for a cell arrest and a possible differentiation. All tested carcinoma cells, HepG2 hepatocarcinoma, HT1376 bladder carcinoma and C33A cervix carcinoma, showed this phenomenon.

In the subsequently initiated experiments with HepG2 cells for discrimination between apoptosis and necrosis (indicated by LDH-leakage and loss of cell membrane integrity), after 24 hours we detected no induction of apoptosis even in the highest tested dosage. The extract according to example 4 did not show any effects, neither apoptosis nor cytotoxicity.

Therefore it was decided to conduct an additional experiment with an incubation of the cells for 48 hours. Interestingly, after 48 hours it was found also no induction of apoptosis.

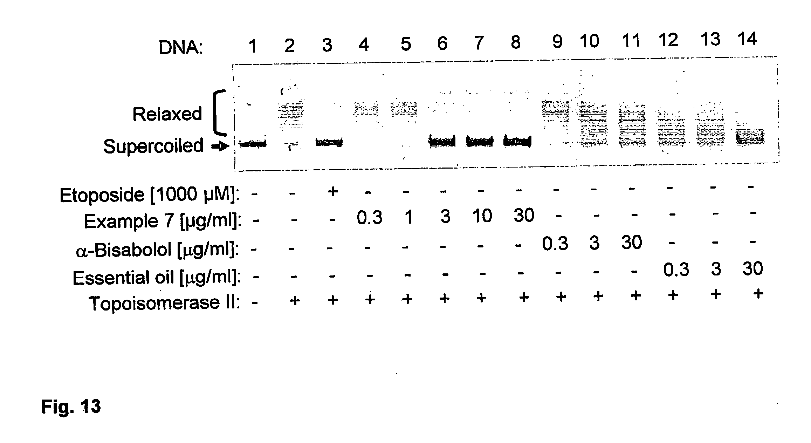

It is obvious from Figure 13 that the extract according to example 7 shows a total inhibition of topoisomerase II activity starting at a concentration of 3 μg/ml. Below the concentration of 3 μg/ml no inhibition is detectable.

α-Bisabolol, one of the main components of the essential oil of a *Chamomilla* plant, did not influence the topoisomerase II activity in all tested concentrations.

The essential oil of *Matricaria recutita L.* hardly inhibited the topoisomerase II activity. Only an incomplete inhibition at a relatively high concentration of 30 μg/ml was observed.

For the purpose of the present invention the terms "containing" or "comprising" shall mean that also additional compounds or components may be present, whereas the term "consist" shall mean that no additional compounds or components than those explicitly mentioned are present.

## 4) Bibliography

1 Toshie H., Noriko N.M., Yoshiyuki A., Mittsuhiro N., Toshiro Y., Naohito O. Granulocyte-Macrophage Colony-Stimulating Factor (GM-CSF) Regulates Cytokine Induction by 1,3-β-D-Glucan SCG in DBA/2 Mice in vitro. J. of IFN and Cytokine Research 24:478-489 (2004).

2 Golenbock D.T., Hampton R.Y., Qureshi N., Takayama K., Raetz C. R. H. (1991) Lipid A-like molecules that antagonize the effect of endotoxins on human monocytes. J. Biol. Chem. 266 (29): 19490-498.

3 Garrelds I.M., van Hal P. Th. W., Haakmat R. C., Hoogsteden H. C., Saxena P. R., Zijlstra F. J.: Time dependent production of cytokines and eicosanoides by human monocytic leukaemia U937 cells; effects of glucocorticosteroids. Mediators of Inflammation: 8, 229-235 (1999).

4 Lindl T. (2000) Zell- und Gewebekultur: Einführung in die Grundlagen sowie ausgewählte Methoden und Anwendungen. 4.überarb. und erw. Auflage - Heidelberg; Berlin: Spectrum, Akad. Verlag (ISBN 3-8274-0803-2).

5 Decker T. & Lohmann-Matthes M.L. (1988) A quick and simple method for the quantification of lactate dehydrogenase release in measurements of cellular cytotoxicity and tumor necrosis factor activity. J. Immunol Methods 15 (1): 61-69.

## Claims

1.  A composition comprising an organic extract of at least one chamomile plant for use in the treatment of an abnormal proliferative and/or viral condition, whereby the extracts are obtainable from Flores tubiformis (tubular flowers) that are free of apigenin and apigenin- glycosides, **characterized in that** the chamomile plant is selected from the group consisting of

    - *Matricaria* species, especially

        -- *Matricaria recutita L*
        -- *Matricaria discoidea DC.*

    - *Anthemis* species, especially

        -- *Anthemis nobilis L.*
        -- *Anthemis arvensis L.*
        -- *Anthemis cotula L.*
        -- *Anthemis tinctoria L.* ;

    or - *Ormenis multicaulis Br.-Bl.* & *Maire,*
    or - *Eriocephalus punctulatus DC,*
    with the proviso that for the treatment of said viral condition the mono/single extract of *Matricaria recutita* (L.) is excluded.

2.  The composition for use according to claim 1, **characterized in that** said composition consists of an organic extract of at least one chamomile plant and occasionally of at least one pharmaceutical and/or dermatological acceptable auxiliary agent.

3.  The composition for use according to one of claims 1 to 2, **characterized in that** the treatment of the abnormal proliferative condition comprises the synchronization and the S-phase arrest of abnormal proliferative mammalian cells, especially of cancer cells.

4.  The composition for use according to claim 3, **characterized in that** said synchronization comprises the induction of ornithine decarboxylase and/or the inhibition of topoisomerases, especially topoisomerase II.

5.  The composition for use according to one of claims 1 to 4, **characterized in that** said organic extracts are obtainable by an extraction with an alcohol, especially an alcohol with 2 to 6 carbon atoms, whereby ethanol is especially preferred, or with a ketone especially a ketone with 3 to 5 carbon atoms, whereby acetone is especially preferred.

6.  The composition for use according to one of claims 1 to 5, **characterized in that** the chamomile plant is *Matricaria recutita L..*

7.  The composition for use according to one of claims 1 to 6, **characterized in that** the plant parts to be extracted are previously subjected to a steam distillation in order to remove the essential oils.

8.  The composition for use according to one of claims 1 to 7, **characterized in that** the primary liquid organic extract is subjected to a molecular weight filtration in order to collect components having a molecular weight of less than 10 000 Dalton and to remove components having a molecular weight of more than 10 000 Dalton, whereby the obtained liquid extract fraction is occasionally processed into a dry powder.

9.  The composition for use according to one of claims 1 to 7, **characterized in that** the primary liquid organic extract is subjected to a molecular weight filtration in order to collect components having a molecular weight of more than 10 000 Dalton and to remove components having a molecular weight of less than 10 000 Dalton, whereby the obtained liquid extract fraction is occasionally processed into a dry powder.

**10.** The composition for use according to one of claims 1 to 9, **characterized in that** it contains at least one pharmaceutical and/or dermatological acceptable auxiliary agent

**11.** The composition for use according to one of claims 1 to 10, **characterized in that** said treatment comprises the simultaneous or sequential administration of this composition and of at least one anti-tumor agent.

**12.** The composition for use according to claim 11, **characterized in that** said treatment comprises

- the administration of this composition during at least 2 days, preferably during at least 5 days, in order to synchronize said abnormal proliferative mammalian cells, especially said cancer cells, and to arrest them in the S-phase, and then
- this administration is stopped at least 6 hours, preferably at least 24 hours, before the start of the administration of at least one anti-tumor agent,

whereby

- during the administration of said anti-tumor agent this composition is not administered, and whereby
- during the following wash-out phase of said anti-tumor agent this composition is also not administered.

**13.** The composition for use according to claim 12, **characterized in that** said treatment cycle is repeated at least twice, preferably at least 3 times.

**14.** The composition for use according to one of claims 11 to 13, **characterized in that** said composition consists of an organic extract of at least one chamomile plant and of at least one anti-tumor agent, and occasionally of at least one pharmaceutical and/or dermatological acceptable auxiliary agent.

**15.** The composition for use according to one of claims 11 to 14, **characterized in that** said anti-tumor agent is of natural, semi-synthetic or synthetic origin.

**16.** The composition for use according to one of claims 11 to 15, **characterized in that** said anti-tumor agent is selected from the group consisting of

- antimetabolites,
- DNA-alkylating agents,
- mitosis inhibitors, and
- DNA-intercalating agents.

**17.** The composition for use according to one of claims 1 to 16, **characterized in that** the primary extract contains at least one water-soluble contaminant having lipid groups which is removed by the following steps:

(i) contacting the composition with a lipophilic component that forms a complex with the contaminant;
(ii) a first removal step of removing material having a particle size larger than the complex formed in step (i); and
(iii) a second removal step of removing the complex formed in step (i).

**18.** The composition for use according to claim 17, **characterized in that** the contaminant is an endotoxin, preferably selected from the group consisting of fragments of the cell wall or fragments of molecules constituting the cell wall of Gram negative bacteria, for example selected from the group consisting of lipopolysaccharides and carbohydrates having protein groups, in particular glycoproteins having at least one lipid chain.

**19.** The composition for use according to one of claims 17 to 18, **characterized in that** the endotoxin has a molecular weight in excess of 10 000 Dalton, preferably in excess of 5 000 Dalton, yet more preferably in excess of 1 000 Dalton and most preferably in excess of 500 Dalton.

**20.** The composition for use according to one of claims 17 to 19, **characterized in that** the lipophilic component is an oil, preferably a fatty oil.

**21.** The composition for use according to one of claims 17 to 20, **characterized in that** step (iii) comprises ultrafiltration, preferably using a filter having a pore size in the range from 0.001 to 0.02 $\mu$m, more preferably from 0.001 to 0.01 $\mu$m.

22. The composition for use according to one of claims 1 to 21, **characterized in that** the extract is free or essentially free of endotoxins, whereby the extract contains endotoxins in an amount of not more than 100 EU/ml - endotoxin units per ml according to European Pharmacopeia -, preferably not more than 50 EU/ml, for example not more than 25 EU/ml.

23. The composition for use according to one of claims 1 to 22, **characterized in that** the treatment is realized by injection of the composition or by oral, rectal or topical administration.

24. Use of a composition comprising an organic extract of at least one chamomile plant for the preparation of a medicament for the treatment of an abnormal proliferative and or viral condition, whereby the extracts are obtainable from Flores tubiformis (tubular flowers) that are free of apigenin and apigenin- glycosides, **characterized in that** the chamomile plant is selected from the group consisting of

   - *Matricaria* species, especially

       -- *Matricaria recutita L*
       -- *Matricaria discoidea DC.*

   - *Anthemis* species, especially

       -- *Anthemis nobilis L.*
       -- - *Anthemis arvensis L.*
       -- *Anthemis cotula L.*
       -- *Anthemis tinctoria L.*,

   or - *Ormenis multicaulis Br.-Bl & Maire,*
   or - *Eriocephalus punctulatus DC,*
   with the proviso that for the treatment of said viral condition the mono/single extract of *Matricaria recutita* (L.) is excluded.

25. Use according to claim 24, **characterized in that** the composition is such a composition as defined in one of claims 2 to 23.


**Patentansprüche**

1. Zubereitung, einen organischen Auszug aus mindestens einer Kamillenpflanze enthaltend, zur Anwendung in der Behandlung eines Leidens mit abnormer Wucherung oder einer Viruserkrankung, wobei die Auszüge aus flores tubiformis (Röhrenblüten), die kein Apigenin und Apigeninglykoside enthalten, gewonnen werden können, **dadurch gekennzeichnet, dass** die Kamillenpflanze aus der Gruppe gewählt wurde, die aus

   - *Matricaria*-Arten, insbesondere
   - *Matricaria recutita L.*
   - *Matricaria discoidea DC*.
   - *Anthemis-Arten,* insbesondere
   - *Anthemis nobilis L.*
   - *Anthemis arvensis* L.
   - *Anthemis cotula L.*
   - *Anthemis tinctoria L.*

   oder - *Ormenis multicaulis* Br.-Bl. & Maire,
   oder - *Eriocephalus punctulatus* DC,
   besteht, unter der Bedingung, dass zur Behandlung der genannten Viruserkrankung der Einzelauszug von *Matricaria recutita* (L.) ausgeschlossen ist.

2. Zubereitung zur Anwendung nach Patentanspruch 1, **dadurch gekennzeichnet, dass** die genannte Zubereitung aus einem organischen Auszug aus mindestens einer Kamillenpflanze und eventuell mindestens einem pharmazeutisch und dermatologisch akzeptablen Zusatzstoff besteht.

3. Zubereitung zur Anwendung nach einem der Patentansprüche 1 bis 2, **dadurch gekennzeichnet, dass** die Behandlung des Leidens mit abnormer Wucherung die Synchronisation und die Blockierung der S-Phase abnorm wuchernder Säugetierzellen, insbesondere von Krebszellen, umfasst.

4. Zubereitung zur Anwendung nach Patentanspruch 3, **dadurch gekennzeichnet, dass** die genannte Synchronisation die Zugabe von Ornithindecarboxylase und oder die Inhibition von Topoisomerasen, insbesondere von Topoisomerase II, umfasst.

5. Zubereitung zur Anwendung nach einem der Patentansprüche 1 bis 4, **dadurch gekennzeichnet, dass** die genannten organischen Auszüge durch alkoholischen Auszug gewonnen werden können, insbesondere mit einem Alkohol mit 2 bis 6 Kohlenstoffatomen, wobei Ethanol besonders bevorzugt wird, oder mit einem Keton, insbesondere einem Keton mit 3 bis 5 Kohlenstoffatomen, wobei Azeton besonders bevorzugt wird.

6. Zubereitung zur Anwendung nach einem der Patentansprüche 1 bis 5, **dadurch gekennzeichnet, dass** die Kamillenpflanze *Matricaria recutita L.* ist.

7. Zubereitung zur Anwendung nach einem der Patentansprüche 1 bis 6, **dadurch gekennzeichnet, dass** die zu extrahierenden Pflanzenteile zunächst einer Dampfdistillation unterworfen werden, um die essentiellen Öle zu entfernen.

8. Zubereitung zur Anwendung nach einem der Patentansprüche 1 bis 7, **dadurch gekennzeichnet, dass** der flüssige organische Rohauszug einer Molekulargewichtsfiltration unterworfen wird, um Bestandteile zu gewinnen, die ein Molekulargewicht von weniger als 10.000 Dalton haben, und Bestandteile zu entfernen, die ein Molekulargewicht von mehr als 10.000 Dalton haben, wobei die gewonnene Fraktion des flüssigen Auszugs eventuell. zu einem trockenen Pulver verarbeitet wird.

9. Zubereitung zur Anwendung nach einem der Patentansprüche 1 bis 7, **dadurch gekennzeichnet, dass** der flüssige organische Rohauszug einer Molekulargewichtsfiltration unterworfen wird, um Bestandteile zu gewinnen, die ein Molekulargewicht von mehr als 10.000 Dalton haben und Bestandteile zu entfernen, die ein Molekulargewicht von weniger als 10.000 Dalton haben, wobei die gewonnene Fraktion des flüssigen Auszugs eventuell zu einem trockenen Pulver verarbeitet wird.

10. Zubereitung zur Anwendung nach einem der Patentansprüche 1 bis 9, **dadurch gekennzeichnet, dass** sie mindestens einen pharmazeutisch und/oder dermatologisch akzeptablen Zusatzstoff enthält.

11. Zubereitung zur Anwendung nach einem der Patentansprüche 1 bis 10, **dadurch gekennzeichnet, dass** die genannte Behandlung die gleichzeitig oder nacheinander erfolgende Verabreichung dieser Zubereitung und mindestens eines Tumormittels umfasst.

12. Zubereitung zur Anwendung nach Patentanspruch 11, **dadurch gekennzeichnet, dass** die genannte Behandlung umfasst:

- Verabreichung dieser Zubereitung während mindestens 2 Tagen, vorzugsweise mindestens 5 Tagen, um die genannten abnorm wuchernden Säugetierzellen, insbesondere die genannten Krebszellen, zu synchronisieren und sie in der S-Phase zu blockieren, und dann
- wird diese Verabreichung mindestens 6 Stunden, vorzugsweise mindestens 24 Stunden, abgesetzt, bevor die Verabreichung des mindestens einen Tumormittels begonnen wird,

wobei

- diese Zubereitung während der Verabreichung des genannten Tumormittels nicht verabreicht wird, und wobei
- in der folgenden Ausspülphase des genannten Tumormittels diese Zubereitung ebenfalls nicht verabreicht wird.

13. Zubereitung zur Anwendung nach Patentanspruch 12, **dadurch gekennzeichnet, dass** der genannte Behandlungszyklus mindestens zweimal, vorzugsweise mindestens dreimal wiederholt wird.

14. Zubereitung zur Anwendung nach einem der Patentansprüche 11 bis 13, **dadurch gekennzeichnet, dass** die genannte Zubereitung aus einem organischen Auszug mindestens einer Kamillenpflanze und mindestens einem Tumormittel besteht und eventuell einem pharmazeutisch und/oder dermatologisch akzeptablen Zusatzstoff.

**15.** Zubereitung zur Anwendung nach einem der Patentansprüche 11 bis 14, **dadurch gekennzeichnet, dass** das genannte Tumormittel natürlichen, halbsynthetischen oder synthetischen Ursprungs ist.

**16.** Zubereitung zur Anwendung nach einem der Patentansprüche 11 bis 15, **dadurch gekennzeichnet, dass** das genannte Tumormittel aus der Gruppe gewählt wurde, bestehend aus

- Antimetaboliten,
- DNS-Alkylantien,
- Mitoseinhibitoren und
- DNS-Interkalantien.

**17.** Zubereitung zur Anwendung nach einem der Patentansprüche 1 bis 16, **dadurch gekennzeichnet, dass** der Rohauszug mindestens einen wasserlöslichen Schmutzstoff enthält, der über Lipidgruppen verfügt, der in den folgenden Schritten entfernt wird:

(i) Kontaktieren der Zubereitung mit einer lipophilen Komponente, die mit dem Schmutzstoff einen Komplex bildet,
(ii) einem ersten Entfernungsschritt zum Beseitigen von Material, das eine Teilchengröße größer als der in Schritt (i) gebildete Komplex aufweist, und
(iii) einem zweiten Entfernungsschritt zum Beseitigen des in Schritt (i) gebildeten Komplexes.

**18.** Zubereitung zur Anwendung nach Patentanspruch 17, **dadurch gekennzeichnet, dass** der Schmutzstoff ein Endotoxin ist, vorzugsweise gewählt aus der Gruppe, bestehend aus Fragmenten der Zellwand oder Fragmenten der Moleküle, die die Zellwand bilden, gramnegativer Bakterien, beispielsweise gewählt aus der Gruppe, bestehend aus Lipopolysacchariden und Kohlenhydraten mit Proteingruppen, insbesondere Glykoproteinen mit mindestens einer Lipidkette.

**19.** Zubereitung zur Anwendung nach einem der Patentansprüche 17 bis 18, **dadurch gekennzeichnet, dass** das Endotoxin ein Molekulargewicht von mehr als 10.000 Dalton hat, vorzugsweise von mehr als 5.000 Dalton und insbesondere von mehr als 1.000 Dalton und besonders bevorzugt von mehr als 500 Dalton.

**20.** Zubereitung zur Anwendung nach einem der Patentansprüche 17 bis 19, **dadurch gekennzeichnet, dass** die lipophile Komponente ein Öl ist, vorzugsweise ein fettes Öl.

**21.** Zubereitung zur Anwendung nach einem der Patentansprüche 17 bis 20, **dadurch gekennzeichnet, dass** Schritt (iii) Ultrafiltration umfasst, vorzugsweise unter Verwendung eines Filters mit einer Porengröße in der Größenordnung von 0,001 bis 0,02 $\mu$m, stärker bevorzugt von 0,001 bis 0,01 $\mu$m.

**22.** Zubereitung zur Anwendung nach einem der Patentansprüche 1 bis 21, **dadurch gekennzeichnet, dass** der Auszug von Endotoxinen frei ist oder im Wesentlichen frei ist, wobei der Auszug Endotoxine in einer Menge von nicht mehr als 100 EU/ml

- Endotoxineinheiten pro ml nach Pharmacopoea Europaea - enthält, vorzugsweise nicht mehr als 50 EU/ml, beispielsweise nicht mehr als 25 EU/ml.

**23.** Zubereitung zur Anwendung nach einem der Patentansprüche 1 bis 22, **dadurch gekennzeichnet, dass** die Behandlung durch Injektion der Zubereitung oder durch orale, rectale oder lokale Verabreichung erfolgt.

**24.** Verwendung einer Zubereitung, einen organischen Auszug aus mindestens einer Kamillenpflanze enthaltend, bei der Zubereitung eines Medikaments zur Behandlung eines Leidens mit abnormer Wucherung oder einer Viruserkrankung, wobei die Auszüge aus flores tubiformis (Röhrenblüten), die kein Apigenin und Apigeninglykoside enthalten, gewonnen werden können, **dadurch gekennzeichnet, dass** die Kamillenpflanze aus der Gruppe gewählt wurde, die aus

- *Matricaria*-Arten, insbesondere
- *Matricaria recutita L.*

*- Matricaria discoidea DC.*
*- Anthemis*-Arten, insbesondere
*- Anthemis nobilis L.*
*- Anthemis arvensis* L.,
*- Anthemis cotula L.,*
*- Anthemis tinctoria L.*

oder - *Ormenis multicaulis* Br.-Bl. & Maire,
oder - *Eriocephatus punctulatus* DC,
besteht, unter der Bedingung, dass zur Behandlung der genannten Viruserkrankung der Einzelauszug von *Matricaria recutita* (L.) ausgeschlossen ist.

25. Anwendung nach Patentanspruch 24, **dadurch gekennzeichnet, dass** die Zubereitung eine Zubereitung nach einem der Patentansprüche 2 bis 23 ist.

**Revendications**

1. Composition comprenant un extrait organique d'au moins une plante de camomille pour utilisation dans le traitement d'une affection virale et/ou d'un état de prolifération anormale, les extraits pouvant être obtenus à partir des Flores tubiformis (fleur d'Eubyla) qui sont exemptes de glycosides d'apigénine, **caractérisée en ce que** la plante de camomille est sélectionnée dans le groupe composé de :

   *- espèce Matricaria*, spécialement
   *- Matricaria recutita L.,*
   *- Matricaria discoidea DC,*
   *- espèce Anthemis,* spécialement
   *- Anthémis nobilis L.,*
   *- Anthémis arvensis L.,*
   *- Anthemis cotula L.,*
   *- Anthémis tinctoria L.,*
   - ou *Ormenis multicaulis Br.-Bl.* & *Maire,*
   - ou *Eriocephalus punctulatus DC,*

   à condition que pour le traitement de ladite affection virale l'extrait mono/unique de *Matricaria recutita* (*L.*) est exclu.

2. Composition pour utilisation selon la revendication 1, **caractérisée en ce que** ladite composition consiste en un extrait organique d'au moins une plante de camomille et parfois en au moins un agent auxiliaire pharmaceutique et/ou dermatologique acceptable.

3. Composition pour utilisation selon l'une des revendications 1 à 2, **caractérisée en ce que** le traitement de l'état de prolifération anormale comprend la synchronisation et l'arrêt de la phase S de cellules mammaires prolifératives anormales, en particulier de cellules cancéreuses.

4. Composition pour utilisation selon la revendication 3, **caractérisée en ce que** ladite synchronisation comprend l'induction de décarboxylase d'ornithine et/ou l'inhibition de topoisomérase, en particulier de topoisomérase II.

5. Composition pour utilisation selon l'une des revendications 1 à 4, **caractérisée en ce que** lesdits extraits organiques peuvent être obtenus par extraction avec un alcool en particulier un alcool avec 2 à 6 atomes de carbone, l'éthanol étant particulièrement préféré ou avec un cétone, en particulier un cétone avec 3 à 5 atomes de carbone, l'acétone étant particulièrement préféré.

6. Composition pour utilisation selon l'une des revendications 1 à 5, **caractérisée en ce que** la plante de camomille est *Matricaria recutita L..*

7. Composition pour utilisation selon l'une des revendications 1 à 6, **caractérisée en ce que** les parties de plante à extraire sont auparavant soumises à une distillation à la vapeur pour enlever les huiles essentielles.

8. Composition pour utilisation selon l'une des revendications 1 à 7, **caractérisée en ce que** l'extrait organique liquide primaire est soumis à une filtration du poids moléculaire pour recueillir des composants qui ont un poids moléculaire inférieur à 10000 Dalton et pour enlever les composants ayant un poids moléculaire supérieur à 10000 Dalton, la fraction d'extrait liquide obtenue étant parfois traitée en une poudre sèche.

9. Composition pour utilisation selon l'une des revendications 1 à 7, **caractérisée en ce que** l'extrait organique liquide primaire est soumis à une filtration du poids moléculaire pour recueillir des composants qui ont un poids moléculaire supérieur à 10000 Dalton et pour enlever les composants ayant un poids moléculaire inférieur à 10000 Dalton, la fraction d'extrait liquide obtenue étant parfois traitée en une poudre sèche.

10. Composition pour utilisation selon l'une des revendications 1 à 9, **caractérisée en ce qu'**elle contient au moins un agent auxiliaire pharmaceutique et/ou dermatologique acceptable.

11. Composition pour utilisation selon l'une des revendications 1 à 10, **caractérisée en ce que** ledit traitement comprend l'administration simultanée ou séquentielle de cette composition et d'au moins un agent anti-tumoral.

12. Composition pour utilisation selon la revendication 11, **caractérisée en ce que** ledit traitement comprend :

- l'administration de cette composition pendant au moins 2 jours, de préférence pendant au moins 5 jours, pour synchroniser lesdites cellules mammaires prolifératives anormales, en particulier lesdites cellules cancéreuses, et pour les arrêter dans la phase S et ensuite
- cette administration est arrêtée pendant au moins 6 heures, de préférence au moins 24 heures, avant le début de l'administration d'au moins un agent anti-tumoral,

cependant que

- pendant l'administration dudit agent anti-tumoral cette composition n'est pas administrée et cependant que
- pendant la phase suivante de rinçage dudit agent anti-tumoral cette composition n'est pas administrée non plus.

13. Composition pour utilisation selon la revendication 12, **caractérisée en ce que** ledit cycle de traitement est répété au moins deux fois, de préférence au moins 3 fois.

14. Composition pour utilisation selon l'une des revendications 11 à 13, **caractérisée en ce que** ladite composition consiste en un extrait organique d'au moins une plante de camomille et d'au moins un agent anti-tumoral et parfois d'au moins un agent auxiliaire pharmaceutique et/ou dermatologique acceptable.

15. Composition pour utilisation selon l'une des revendications 11 à 14, **caractérisée en ce que** ledit agent anti-tumoral est d'origine naturelle, semi-synthétique ou synthétique.

16. Composition pour utilisation selon l'une des revendications 11 à 15, **caractérisée en ce que** ledit agent anti-tumoral est sélectionné dans le groupe composé :

- des antimétabolites,
- des agents d'alkylation de l'ADN,
- des inhibiteurs de mitose,
- des agents intercalant d'ADN.

17. Composition pour utilisation selon l'une des revendications 1 à 16, **caractérisée en ce que** l'extrait primaire contient au moins un contaminant soluble dans l'eau qui a des groupes lipides qui sont enlevés par les étapes suivantes :

(i) mise en contact de la composition avec un composant lipophile qui forme un complexe avec le contaminant ;
(ii) une première étape d'enlèvement de matière ayant une dimension de particules supérieure au complexe formé à l'étape (i) et
(iii) une seconde étape d'enlèvement du complexe formé à l'étape (i).

18. Composition pour utilisation selon la revendication 17, **caractérisée en ce que** le contaminant est une endotoxine, de préférence sélectionnée dans le groupe composé de fragments de la paroi cellulaire ou de fragments de molécules constituant la paroi cellulaire de bactéries Gramm-négatives, par exemple sélectionnée dans le groupe composé des lipopolysaccharides et des hydrates de carbone ayant des groupes de protéines, en particulier des glycoprotéines ayant au moins une chaîne de lipides.

19. Composition pour utilisation selon l'une des revendications 17 à 18, **caractérisée en ce que** l'endotoxine a un poids moléculaire supérieur à 10000 Dalton, de préférence supérieur à 5000 Dalton, de manière encore plus préférée supérieur à 1000 Dalton et de manière la plus préférée supérieur à 500 Dalton.

20. Composition pour utilisation selon l'une des revendications 17 à 19, **caractérisée en ce que** le composant lipophile est une huile, de préférence une huile grasse.

21. Composition pour utilisation selon l'une des revendications 17 à 20, **caractérisée en ce que** l'étape (iii) comprend l'ultrafiltration, de préférence en utilisant un filtre ayant une dimension de pore de l'ordre de 0,001 à 0,02 $\mu$m, de manière plus préférée de 0,001 à 0,01 $\mu$m.

22. Composition pour utilisation selon l'une des revendications 1 à 21, **caractérisée en ce que** l'extrait est exempt ou essentiellement exempt d'endotoxines, cependant que l'extrait contient des endotoxines en une quantité qui ne dépasse pas plus de 100 EU/ml - unités d'endotoxine par ml selon la pharmacopée européenne - de préférence qui ne dépasse pas plus de 50 EU/ml, par exemple qui ne dépasse pas plus de 25 EU/ml.

23. Composition pour utilisation selon l'une des revendications 1 à 22, **caractérisée en ce que** le traitement est réalisé par injection de la composition ou par administration orale, rectale ou topique.

24. Utilisation d'une composition comprenant un extrait organique d'au moins une plante de camomille pour la préparation d'un médicament pour le traitement d'une affection virale et/ou d'un état de prolifération anormale, les extraits pouvant être obtenus à partir des Flores tubiformis (fleur d'Eubyla) qui sont exemptes de glycosides d'apigénine, **caractérisée en ce que** la plante de camomille est sélectionnée dans le groupe composé de :

  - *espèce Matricaria,* spécialement
  - *Matricaria recutita L.,*
  - *Matricaria discoidea DC,*
  - *espèce Anthemis,* spécialement
  - *Anthemis nobilis L.,*
  - *Anthemis arvensis L.,*
  - *Anthémis cotula L.,*
  - *Anthémis tinctoria L.,*
  - ou *Ormenis multicaulis Br.-Bl. & Maire,*
  - ou *Eriocephalus punctulatus DC,*

à condition que pour le traitement de ladite affection virale l'extrait mono/unique de *Matricaria recutita (L.)* est exclu.

25. Utilisation selon la revendication 24, **caractérisée en ce que** la composition est une composition telle que défini dans l'une des revendications 2 à 23.

**Fig. 1**

**Fig. 2**

EP 2 288 363 B1

Fig. 3

22

**Fig. 4**

**Fig. 5**

**Fig. 6**

**Fig. 7**

**Fig. 8**

**Fig. 9**

**Fig. 10**

**Fig. 11**

**Fig. 12**

EP 2 288 363 B1

| DNA: | 1 | 2 | 3 | 4 | 5 | 6 | 7 | 8 | 9 | 10 | 11 | 12 | 13 | 14 |
|---|---|---|---|---|---|---|---|---|---|---|---|---|---|---|

Relaxed [

Supercoiled →

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Etoposide [1000 µM]: | - | - | + | - | - | - | - | - | - | - | - | - | - | - |
| Example 7 [µg/ml]: | - | - | - | 0.3 | 1 | 3 | 10 | 30 | - | - | - | - | - | - |
| α-Bisabolol [µg/ml]: | - | - | - | - | - | - | - | - | 0.3 | 3 | 30 | - | - | - |
| Essential oil [µg/ml]: | - | - | - | - | - | - | - | - | - | - | - | 0.3 | 3 | 30 |
| Topoisomerase II: | - | + | + | + | + | + | + | + | + | + | + | + | + | + |

**Fig. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03101479 A1
- WO 2007057651 A1
- WO 0113929 A1

- WO 2008146009 A1
- US 6300370 B1

### Non-patent literature cited in the description

- **P.VILAGINÈS ; P. DELAVEAU ; R. VILAGINÈS.** Inhibition de la replication du poliovirus par un extrait de Matricaria chamomilla (L.). *Comptes Rendus de l'Academie des Sciences, Série III,* 1985, vol. 301 (6), 289-294
- **GRATZNER, H. G.** *Science,* 1982, vol. 218, 474-475
- **TOSHIE H. ; NORIKO N.M. ; YOSHIYUKI A. ; MITTSUHIRO N. ; TOSHIRO Y. ; NAOHITO O.** Granulocyte-Macrophage Colony-Stimulating Factor (GM-CSF) Regulates Cytokine Induction by 1,3-β-D-Glucan SCG in DBA/2 Mice in vitro. *J. of IFN and Cytokine Research,* 2004, vol. 24, 478-489
- **GOLENBOCK D.T. ; HAMPTON R.Y. ; QURESHI N. ; TAKAYAMA K. ; RAETZ C. R. H.** Lipid A-like molecules that antagonize the effect of endotoxins on human monocytes. *J. Biol. Chem.,* 1991, vol. 266 (29), 19490-498

- **GARRELDS I.M. ; VAN HAL P. TH. W. ; HAAKMAT R. C. ; HOOGSTEDEN H. C. ; SAXENA P. R. ; ZIJLSTRA F. J.** Time dependent production of cytokines and eicosanoides by human monocytic leukaemia U937 cells; effects of glucocorticosteroids. *Mediators of Inflammation,* 1999, vol. 8, 229-235
- Zell- und Gewebekultur: Einführung in die Grundlagen sowie ausgewählte Methoden und Anwendungen. **LINDL T.** Spectrum. Akad. Verlag, 2000
- **DECKER T. ; LOHMANN-MATTHES M.L.** A quick and simple method for the quantification of lactate dehydrogenase release in measurements of cellular cytotoxicity and tumor necrosis factor activity. *J. Immunol Methods,* 1988, vol. 15 (1), 61-69